# DEMANDE DE BREVET EUROPEEN

(11) **EP 1 634 622 A1**
(43) Date de publication de la demande: **15.03.2006**
(21) Numéro de dépôt: 05291719.2
(22) Date de dépôt: 11.08.2005
(51) Int. Cl.: A61Q 5/10

(54) **Procédé de coloration de fibres kératiniques à partir d'une composition comprenant un colorant hydrophobe particulier**

(30) Priorité: 16.08.2004 FR 0451856
(71) Demandeur: L'OREAL, 75008 Paris (FR)
(72) Inventeur: De Boni, Maxime, 75012 Paris (FR); Kravtchenko, Sylvain, 92600 Asnières (FR); Lagrange, Alain, 77700 Coupvray (FR)
(74) Mandataire: Fevrier, Murielle Françoise E.

(57) **Abrégé**

La présente invention a pour objet un procédé de coloration des fibres kératiniques qui comprend l'application sur les fibres kératiniques d'une composition comprenant au moins un colorant direct hydrophobe dont le logP est supérieur à 2 dans un milieu de coloration hydroalcoolique contenant au plus 60 % d'eau, l'application étant effectuée à une température supérieure ou égale à 60°C.

Le procédé de l'invention permet d'obtenir des nuances variant du pastel à des colorations intenses. De plus, la coloration obtenue permet d'atteindre voire de dépasser la ténacité de la coloration d'oxydation.

## Description

L'invention a pour objet un procédé de coloration de fibres kératiniques à partir d'une composition tinctoriale comprenant dans un milieu approprié un colorant hydrophobe particulier.

Depuis longtemps, de nombreuses personnes cherchent à modifier la couleur de leurs cheveux et en particulier à masquer leurs cheveux blancs.

Pour ce faire, il est connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs, sous l'action d'un agent oxydant, forment dans le cheveu une ou plusieurs espèces colorées.

La variété des molécules mises en jeu au niveau des bases d'oxydation et des coupleurs, permet l'obtention d'une riche palette de couleurs. Les colorations qui en résultent sont permanentes, puissantes, et résistantes aux agents extérieurs, notamment à la lumière, aux intempéries, aux lavages, à la transpiration et aux frottements.

Cependant, la coloration est effectuée à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées ce qui peut entraîner un coiffage ou une mise en forme difficile.

Par ailleurs, il est connu de teindre les fibres kératiniques et en particulier les cheveux humains, avec des compositions de teinture contenant des colorants directs. Les colorants classiques qui sont utilisés sont en particulier des colorants du type nitrés benzèniques, anthraquinoniques, nitropyridiniques, azoïques, xanthéniques, acridiniques, aziniques, triarylméthane ou des colorants naturels. Ces colorants peuvent être non ioniques, anioniques, cationiques ou amphotères. Ces colorants sont des molécules colorées et colorantes ayant une affinité pour les fibres kératiniques.

Les colorants directs sont en général utilisés dans un milieu constitué majoritairement par de l'eau, avec éventuellement une quantité mineure de solvant tel que des alcools. De telles compositions sont par exemple décrites dans les documents EP 1 366 752, EP 1 369 105. Il est aussi connu afin d'améliorer la solubilité dans l'eau des colorants cationiques, dispersés ou solvants par addition d'un tensio-actif anionique comme dans le brevet US 5 593 459.

Ces compositions contenant un ou plusieurs colorants directs sont appliquées sur les fibres kératiniques pendant un temps nécessaire à l'obtention de la coloration désirée, puis rincés.

Les colorations qui en résultent sont des colorations souvent chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Il existe toujours un besoin de développer de nouvelles compositions de teinture directe pour d'obtenir des nuances variées, en particulier dans des nuances pastels et qui présentent une bonne ténacité, notamment aux agents extérieurs tels que la lumière, le shampooing, la sueur. En particulier, il existe un besoin de développer des compositions de coloration permettant d'obtenir des colorations présentant une ténacité proche de la coloration par oxydation sans les inconvénients liés à la présence d'un agent oxydant.

Ce but est atteint avec la présente invention qui a pour objet un procédé de coloration des fibres kératiniques qui comprend l'application sur les fibres kératiniques d'une composition comprenant au moins un colorant direct hydrophobe dont le logP est supérieur à 2 dans un milieu de coloration hydroalcoolique contenant au plus 60 % d'eau, l'application étant effectuée à une température supérieure ou égale à 60°C.

Le procédé de l'invention permet d'obtenir des nuances variant du pastel à des colorations intenses. De plus, la coloration obtenue permet d'atteindre voire de dépasser la ténacité de la coloration d'oxydation. Ainsi, la coloration obtenue est très résistante aux agents extérieurs, notamment aux lavages répétés.

Dans le cadre de l'invention, la valeur du logP représente de façon classique le coefficient de partage du colorant entre l'octanol et l'eau. Le logP peut être calculée selon la méthode décrite dans l'article de Meylan et Howard *« Atom* / *Fragment contribution method for estimating octanol-water partition coefficient »,* J. Pharm. Sci. 84 :83-92, 1995. Cette valeur peut aussi être calculée à partir de nombreux logiciels disponibles sur le marché qui détermine le logP en fonction de la structure d'une molécule. A titre d'exemple, on peut citer le logiciel Epiwin de l'agence de l'environnement des Etats-Unis.

Les colorants directs qui peuvent être utilisés dans la composition utile dans la présente invention sont des colorants hydrophobes connus de la technique qui présentent un logP supérieur à 2. A titre d'exemple, on peut citer :

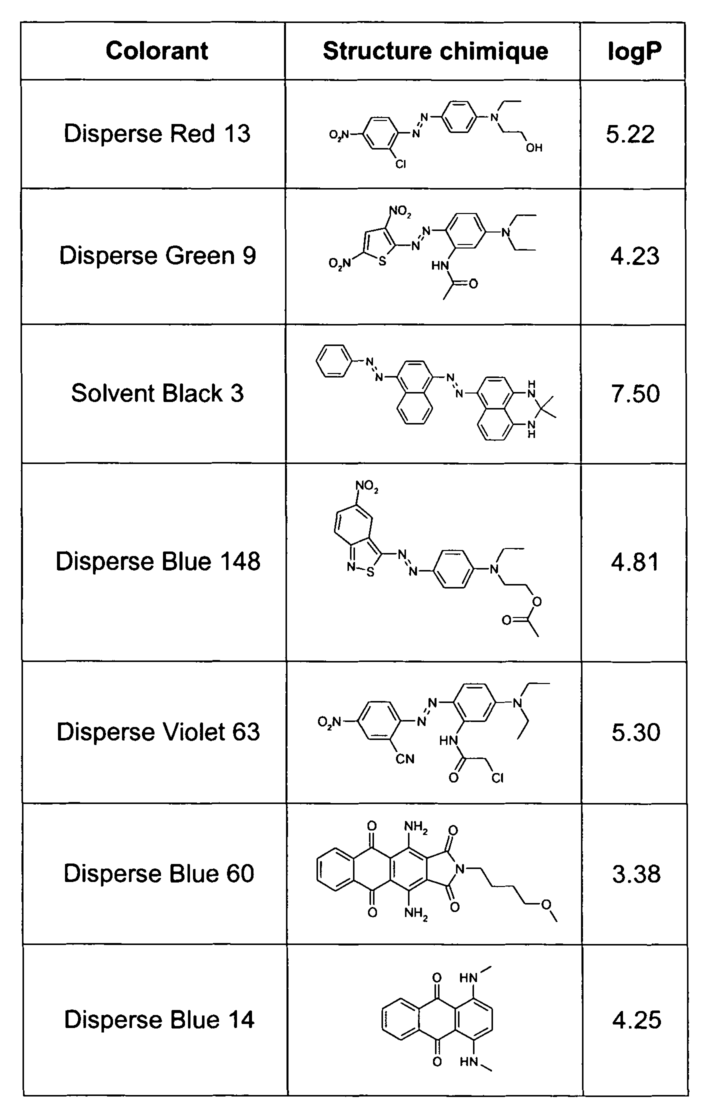

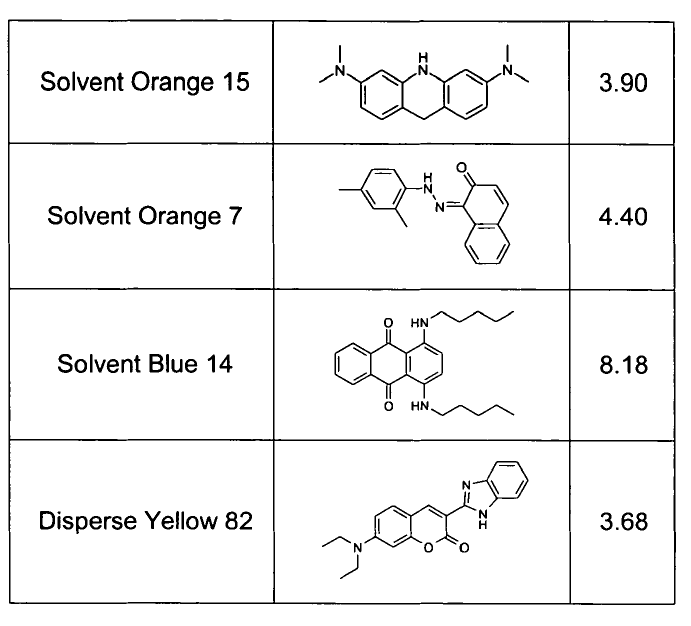

Selon un mode de réalisation particulier, le logP du colorant utile dans l'invention est supérieur à 4.

Le ou les colorants directs présentant un logP supérieur à 2 peuvent être présents dans la composition dans des quantités comprises entre 0,001 à 5 % en poids environ du poids total de la composition prête à l'emploi.

A titre d'alcool pouvant être utilisé dans le milieu de coloration hydroalcoolique, on peut notamment citer les alcanols inférieurs en C₁-C₆, les polyols et éthers de polyols possédant une fonction -OH libre. A titre d'exemple, on peut citer le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, le néopentyl glycol, l'isoprèneglycol ainsi que les alcools aromatiques comme l'alcool benzylique ou le phénoxyéthanol, l'alcool phényléthylique, l'éthanol, l'isopropanol, le n-propanol, le butanol, le n-pentanol, , le 1,2-propanediol, le 1,3-propanediol, le 1-méthoxy 2-propanol, le 1-éthoxy 2-propanediol, les 1,3 et 1,4- butanediol, le 1,2-hexanediol ou un mélange de ces alcools.

La composition contient de préférence 0,1 à 60 % d'eau, préférentiellement de 20 à 60 %, et encore plus préférentiellement de 40 à 55 % d'eau.

Selon un mode de réalisation particulier, la quantité d'alcool est au minimum de 5 %, de préférence comprise entre 10 et 75 %. Selon un mode de réalisation préféré, la quantité d'alcool est comprise entre 30 et 60 %.

Les % sont des pourcentages en poids par rapport au poids total de la composition utile dans le procédé de l'invention.

La composition utile dans le procédé de l'invention peut en outre contenir des colorants directs additionnels différents des colorants directs hydrophobe décrits précédemment. Ces colorants directs additionnels sont par exemple des colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants:
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Acid Violet 43
- Acid Blue 62
- Basic Blue 22
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants directs naturels utilisables selon l'invention, on peut citer la lawsone, la juglone, l'alizarine, la purpurine, l'acide carminique, l'acide kermésique, la spinulosine. On peut également utiliser les extraits ou décoctions contenant ces colorants naturels et notamment les cataplasmes ou extraits à base de henné.

Lorsque cette composition comprend d'autres colorants directs que ceux présentant un logP supérieur à 2, la composition peut contenir jusqu'à 20 % de colorants directs. Selon ce mode particulier de réalisation, la composition de l'invention comprend une quantité totale de colorants directs comprise entre 0,001 à 10% en poids environ.

La composition peut de plus contenir des bases d'oxydations et des coupleurs classiquement utilisés pour la coloration par oxydation.

A titre d'exemple, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, les bases et les coupleurs sont chacun généralement présents en quantité comprise entre 0,001 et 10 % en poids environ du poids total de la composition tinctoriale, de préférence entre 0,005 et 6 %.

La composition peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Pour la teinture des fibres kératiniques humaines, le milieu de coloration est un milieu cosmétique.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le pH de la composition tinctoriale conforme à l'invention est généralement compris entre 2 et 12 environ, et de préférence entre 2 et 7.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 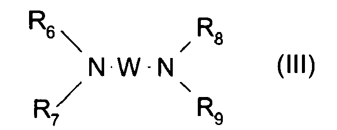 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

Dans le procédé de l'invention, le temps de pause de la composition sur les fibres kératiniques est généralement compris entre 1 à 60 minutes environ, de préférence 10 à 60 minutes environ. Après le temps de pause, les fibres kératiniques sont rincées laissant apparaître des fibres colorées.

La température d'au moins 60°C peut être obtenue par divers moyens tels qu'un sèche-cheveux, un casque ou un fer à friser ou à lisser. Selon un mode de réalisation particulier, la température d'application de la composition est comprise entre 60°C et 250°C, de préférence entre 60 et 100°C.

Lorsque la composition tinctoriale peut contenir un agent oxydant soit pour éclaircir les fibres soit lors de l'utilisation d'une base d'oxydation et/ou d'un coupleur dans la composition. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Les exemples qui suivent servent à illustrer l'invention sans toutefois présenter un caractère limitatif.

### EXEMPLES

### Exemple 1

Une solution eau/éthanol (1:1) contenant 0.3% de Disperse Red 13 est appliquée sur des mèches de cheveux naturels gris contenant 90% de cheveux blancs et sur des mèches de cheveux permanentés gris contenant 90% de cheveux blancs.

Après une application de 30 minutes à 60°C, les mèches sont rincées et séchées. Les mèches ainsi traitées sont colorées en rouge.

## Revendications

1. Procédé de coloration des fibres kératiniques comprenant l'application sur les fibres d'une composition de coloration comprenant au moins un colorant direct hydrophobe dont le logP est supérieur à 2 dans un milieu de coloration hydroalcoolique contenant au plus 60 % d'eau, l'application sur les fibres étant effectuée à une température supérieure ou égale à 60°C.

2. Procédé selon la revendication 1 dans lequel au moins un des colorants directs hydrophobes présente un logP supérieur à 4.

3. Procédé selon la revendication 1 ou 2 dans lequel l'alcool présent dans la composition est choisi parmi les alcanols inférieurs en C₁-C₆, les polyols et éthers de polyols, seuls ou en mélange.

4. Procédé selon la revendication 3 dans lequel l'alcool est choisi parmi le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, , le néopentyl glycol, l'isoprèneglycol ainsi que les alcools aromatiques comme l'alcool benzylique ou le phénoxyéthanol, l'alcool phényléthylique, l'éthanol, l'isopropanol, le n-propanol, le butanol, le n-pentanol, , le 1,2-propanediol, le 1,3-propanediol, le 1-méthoxy 2-propanol, le 1-éthoxy 2-propanediol, les 1,3 et 1,4- butanediol, le 1,2-hexanediol ou un mélange de ces alcools.

5. Procédé selon l'une quelconque des revendications 1 à 4 dans lequel la quantité d'eau dans la milieu de coloration hydroalcoolique est comprise entre 0,1 et 60%.

6. Procédé selon la revendication 5 dans lequel la quantité d'eau est comprise entre 20 et 60 %.

7. Procédé selon la revendication 6 dans lequel la quantité d'eau est comprise entre 40 et 55 %.

8. Procédé selon l'une quelconque des revendications précédentes dans lequel la quantité d'alcool dans le milieu de coloration hydroalcoolique est supérieure ou égale à 5%.

9. Procédé selon la revendication 8 dans lequel la quantité d'alcool est comprise entre 10 et 75 %.

10. Procédé selon la revendication 9 dans lequel la quantité d'alcool est comprise entre 30 et 60 %.

11. Procédé selon l'une quelconque des revendications 1 à 10 dans lequel le ou les colorants directs présentant un logP supérieur à 2 sont présents dans la composition en quantité comprise entre 0,001 à 5 % en poids environ du poids total de la composition.

12. Procédé selon l'une quelconque des revendications précédentes dans lequel la composition comprend un colorant direct additionnel choisi parmi les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

13. Procédé selon l'une quelconque des revendications précédentes dans lequel la composition comprend une base d'oxydation choisie parmi les paraphénylènediamines, les bis-phénylalkylènediamines, les para-aminophénols, les ortho-aminophénols, les bases hétérocycliques et leurs sels d'addition.

14. Procédé selon l'une quelconque des revendications précédentes dans lequel la composition comprend un coupleur choisi parmi les métaphénylènediamines, les méta-aminophénols, les métadiphénols, les coupleurs naphtaléniques, les coupleurs hétérocycliques et leurs sels d'addition.

15. Procédé selon la revendication 13 dans lequel la quantité de bases d'oxydation est comprise entre 0,001 à 10 % en poids environ du poids total de la composition tinctoriale.

16. Procédé selon la revendication 14 dans lequel la quantité de chacun des coupleurs est comprise entre 0,001 et 10 % en poids environ du poids total de la composition tinctoriale.

17. Procédé selon l'une quelconque des revendications 1 à 16 dans lequel la composition comprend de plus un ou plusieurs adjuvants choisis parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, les agents épaississants associatifs polymères anioniques, cationiques, non ioniques et amphotères, les agents antioxydants, les agents de pénétration, les agents séquestrants, les parfums, les tampons, les agents dispersants, les agents de conditionnement, les agents filmogènes, les céramides, les agents conservateurs, les agents opacifiants.

18. Procédé selon la revendication 17 dans lequel les adjuvants sont présents en quantité comprise pour chacun d'eux entre 0,01 et 20 % en poids par rapport au poids de la composition.

19. Procédé selon l'une quelconque des revendications précédentes dans lequel le pH de la composition est compris entre 2 et 12 environ.

20. Procédé selon l'une quelconque des revendications précédentes dans lequel le temps d'application est compris entre 1 et 60 min.

21. Procédé selon l'une quelconque des revendications 1 à 20 dans lequel la température de la composition est comprise entre 60°C et 250°C.

22. Procédé selon la revendication 21 dans lequel la température est comprise entre 60 et 100°C.
